# DEMANDE DE BREVET EUROPEEN

(11) **EP 1 475 076 A1**
(43) Date de publication de la demande: **10.11.2004**
(21) Numéro de dépôt: 04291138.8
(22) Date de dépôt: 04.05.2004
(51) Int. Cl.: A61K 7/09

(54) **Dithiols à fonction amide et leur utilisation pour la transformation de la forme du cheveu**

(30) Priorité: 06.05.2003 FR 0305497
(71) Demandeur: L'OREAL, 75008 Paris (FR)
(72) Inventeur: Philippe, Michel, 91320 Wissous (FR); Andrean, Hervé, 75014 Paris (FR)
(74) Mandataire: Bourdeau, Françoise

(57) **Abrégé**

L'invention a pour objet l'utilisation en cosmétique des dithiols à fonction amide, pour la transformation de la forme des cheveux. Elle vise également les compositions cosmétiques contenant les dithiols à fonction amide, dans un milieu cosmétiquement acceptable.

## Description

L'invention a pour objet l'utilisation en cosmétique de dithiols à fonction amide, pour la transformation de la forme des cheveux. Elle vise également les compositions cosmétiques contenant ces dithiols à fonction amide, dans un milieu cosmétiquement acceptable.

Dans tout le texte, le terme "dithiol" englobe tous les éventuels isomères optiques de ce dernier.

On entend par "fibre kératinique", les cheveux, les cils et les sourcils, les poils et moustaches et surtout les cheveux.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis après avoir de préférence, rincé la chevelure, à reconstituer dans un second temps lesdites liaisons disulfures en appliquant, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou leur décrêpage, les cheveux pouvant être selon les cas, mis sous tension pendant ou en dehors du procédé de déformation.

Les compositions pour réaliser le premier temps d'une opération de permanente se présentent généralement sous forme de lotions, de crèmes, de gels, de poudres à diluer dans un support liquide, et contiennent, un agent réducteur, de préférence un thiol. Parmi ces derniers, ceux couramment utilisés sont la cystéine et l'acide thioglycolique ainsi que ses esters, notamment le monothioglycolate de glycérol. L'acide thioglycolique est particulièrement efficace pour réduire les liaisons disulfures de la kératine et peut être considéré à pH alcalin, notamment sous forme de thioglycolate d'ammonium, comme le composé de référence en permanente. Il présente cependant l'inconvénient de dégager une odeur désagréable. En vue d'y remédier, il est généralement fait usage d'un parfum permettant de masquer les odeurs.

La cystéine produit une odeur beaucoup plus faible que celle de l'acide thioglycolique mais le degré de frisure obtenu est très inférieur et loin d'être satisfaisant. De plus, la cystéine nécessite l'utilisation d'un pH très alcalin.

Le monothioglycolate de glycérol est également très malodorant. Il est, par contre, utilisé à un pH proche de la neutralité, mais ses performances sont notablement inférieures à celles de l'acide thioglycolique.

Diverses études ont été conduites en vue de remédier aux inconvénients de ces agents réducteurs, et à cet effet, il a été proposé l'emploi de nouveaux composés ou systèmes réducteurs. Ainsi, dans les brevets US 2 719 813 et US 2 719 814, il a déjà été proposé d'utiliser un mercaptan et son disulfure, en tant que système réducteur des cheveux, en recommandant plus particulièrement d'ajuster le pH de la composition réductrice entre 8,5 et 9,5. Le brevet US 2 719 813 concerne spécialement une composition réductrice pour permanente formée par du dithiodiglycérol et du thioglycérol et le brevet US 2 719 814, une composition réductrice contenant, en particulier de l'acide thiohydracrylique et dithiohydracrylique.

Par ailleurs, le brevet US 5 460 806 divulgue l'utilisation de bis(2-mercaptoéthyl) sulfone (BMS), en tant qu'agent réducteur dans un procédé capillaire de défrisage ou de frisage des cheveux, sans préciser le pH de mise en oeuvre de solutions réductrices contenant de tels agents réducteurs. Ce composé est très peu soluble dans l'eau et donc de mise en oeuvre pratiquement impossible dans les conditions habituelles de déformation permanente.

Enfin, il est déjà connu, en cosmétique, l'utilisation de certains dithiols. En particulier, la demande de brevet français, déposée par la Demanderesse sous le numéro 0204 352 et, non encore publiée, divulgue un procédé de gainage métallique conférant, aux fibres kératiniques, des propriétés cosmétiques rémanentes aux shampooings. Selon ce procédé, on applique sur les fibres kératiniques une composition contenant au moins un sel métallique, puis on applique une composition contenant au moins un agent réducteur. Parmi ces derniers sont cités les dithiols suivants: BMS (bismercaptoéthylsulfone), DTT (dithiothréitol), DMH (N,N'diméthyl-N,N'-bis(mercaptoacétyl)hydrazine).

Dans cette demande de brevet français, il est indiqué que les fibres kératiniques peuvent être réduites, préalablement à l'application de la composition contenant au moins un sel métallique. Il est prévu que cette réduction peut être effectuée avec l'un des agents réducteurs décrits dans le texte de cette demande, comme les dithiols précédemment cités.

Par ailleurs, il est connu l'utilisation du dithiol BMC contenant deux fonctions amides, pour la réduction de protéines dans WO98/04734.

Néanmoins, les compositions réductrices pour permanentes connues à ce jour ne donnent pas encore entièrement satisfaction, étant donné que les dégradations de la fibre capillaire sont trop importantes.

En outre, lorsqu'une permanente est appliquée sur des cheveux ayant subi une coloration, un très fort décapage de la couleur est observé et la surface des cheveux est fortement dégradée. A l'inverse si une coloration est appliquée sur des cheveux permanentés la couleur obtenue est très différente de la couleur normalement obtenue sur des cheveux naturels non permanentés.

Le problème posé par l'invention est de fournir une composition réductrice, pour la déformation permanente des cheveux, plus efficace que celles qui existent déjà, notamment en termes de degré, de nervosité ou de qualité de frisure, tout en diminuant la dégradation des cheveux, cette composition réductrice devant, en outre, être pratiquement sans effet sur une coloration artificielle des fibres kératiniques.

Pour résoudre ce problème, l'invention propose l'utilisation, pour réduire les liaisons disulfure de la kératine, dans une composition réductrice de déformation de la fibre kératinique, notamment à un pH compris entre 6 et 8, de dithiols de formule 1, 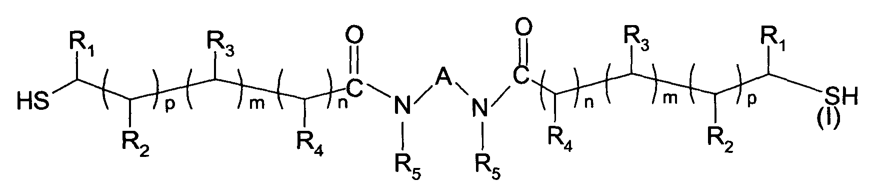 Avec
- n,m,p=0 ou1
- R₁, R₂, R₃, R₄ = H, COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
- A = alkylène en C-1 à C-18, saturé ou non, linéaire ou non, pouvant être interrompu par un ou plusieurs cycles aromatiques ou non aromatiques (5, 6 ou 7 chaînons) ou par un ou plusieurs hétéro-atomes ou fonctions tels 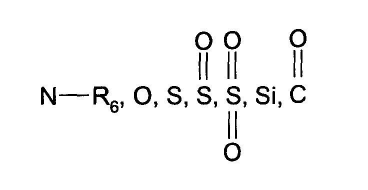 A peut également être représenté par un cycle aromatique ou non aromatique à 5, 6 ou 7 chaînons.
   A peut être substitué par un plusieurs groupements COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
- R₅, R₆ = H, alkyl (C-1 à C-4), acyl (C-1 à C-4), alkyl (C-1 à C-4) oxycarbonyl, alkyl (C-1 à C-4) amino-carbonyl, halogéno.

La liste et la structure des composés préférés sont données ci-après :

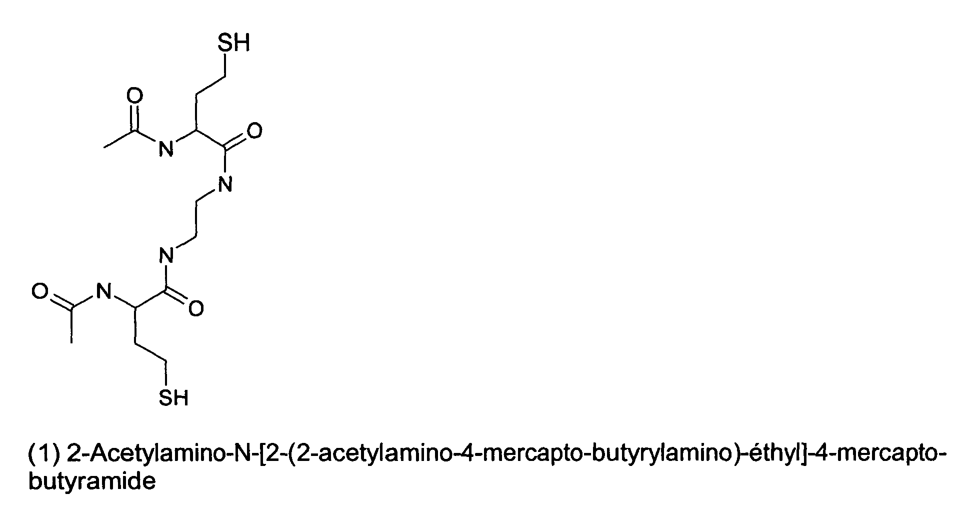

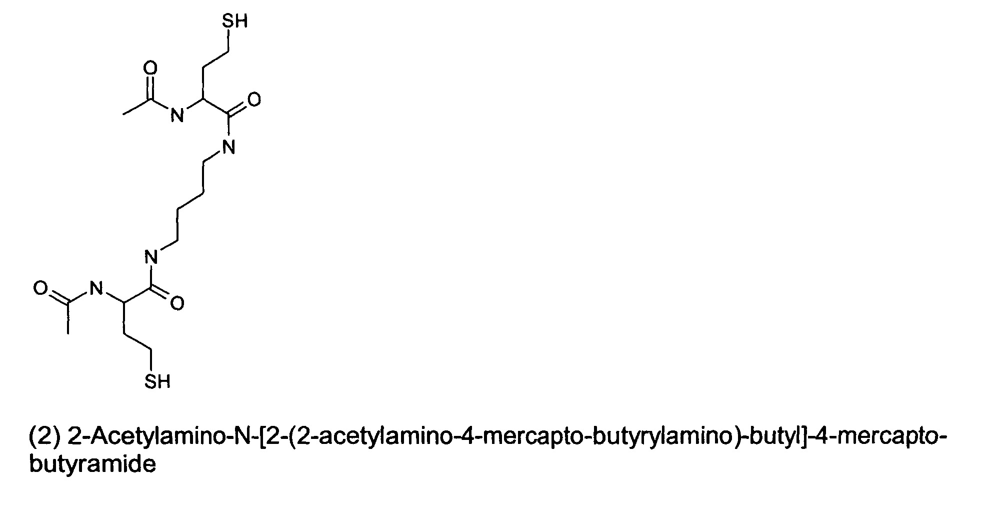

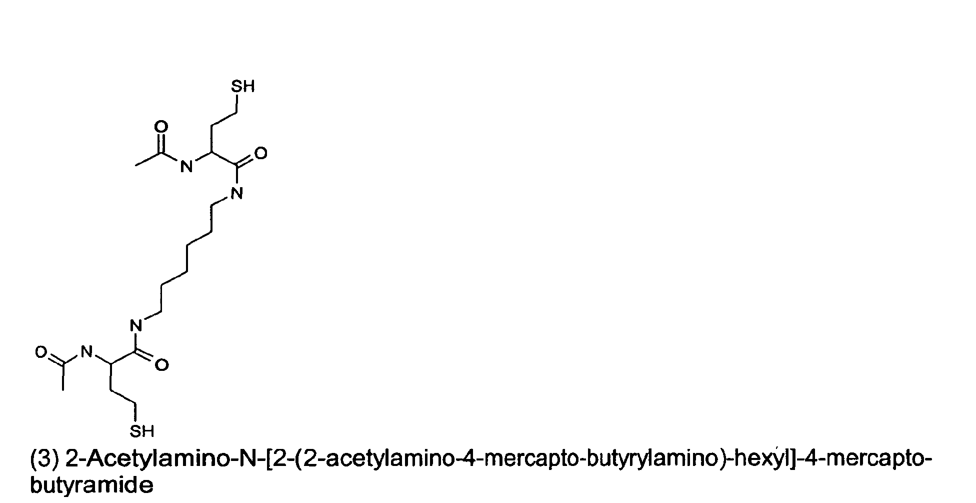

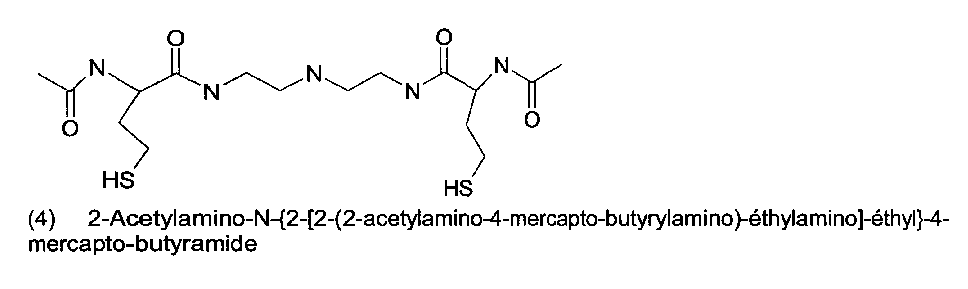

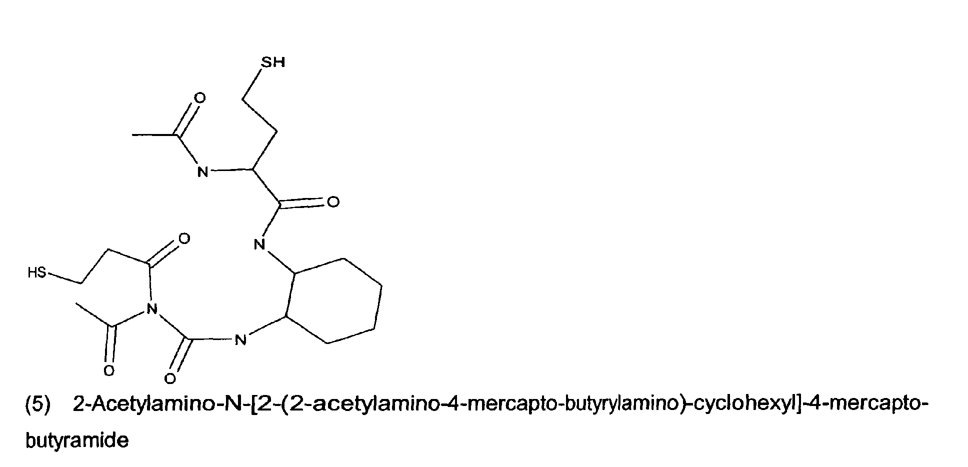

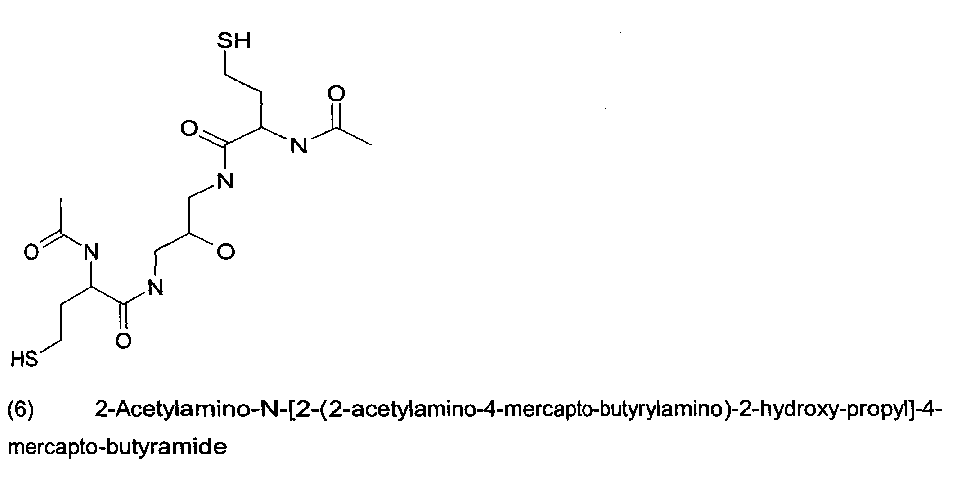

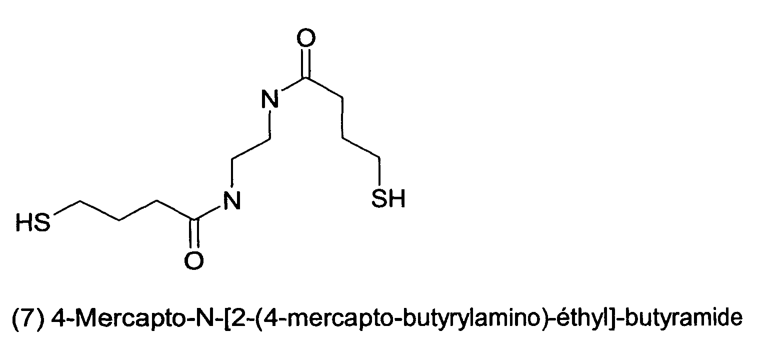

Avantageusement, le choix du pH entre 6,5 et 7,5, et notamment de 7, étant notoirement moins dégradant pour la fibre que des pH basiques, il en résulte un avantage important par rapport à l'utilisation actuelle à pH 8,5 et plus des thiols comme l'acide thioglycolique.

Un autre objet de l'invention concerne un procédé de déformation permanente des cheveux mettant en oeuvre une composition réductrice comprenant au moins un composé de formule (I).

Les composés de formule (I) sont généralement préparés en faisant réagir, sous atmosphère inerte une amine sur une thiolactone, à une température comprise entre 0°C et la température de reflux du solvant pour une durée de 15 minutes à 12 heures.

Un objet de l'invention concerne un procédé de préparation d'un composé de formule (I).

Le procédé conforme à l'invention de déformation permanente des cheveux comprend l'application d'une composition réductrice comprenant, en tant qu'agent réducteur, un composé de formule (I). Les cheveux sont mis en forme en utilisant des moyens mécaniques bien connus de l'homme de l'art, comme des bigoudis, la composition réductrice étant appliquée avant ou après les éventuels moyens de mise en forme des cheveux, et une composition de fixation étant appliquée après la composition réductrice, avec ou sans étape intermédiaire ou subséquente de rinçage ou d'application de composition intermédiaire.

Avantageusement, le procédé selon l'invention comprend l'application d'une composition réductrice définie dans l'une quelconque des revendications 1 à 7 contenant, en tant qu'agent réducteur, un composé de formule (I), les cheveux étant mis en forme par des moyens appropriés; une composition de fixation étant, en outre, appliquée après la composition réductrice, après un éventuel rinçage.

Selon la présente invention, la déformation permanente des cheveux consiste, de préférence, dans une première étape, à réduire les liaisons disulfures de la kératine par application, pendant environ 5 à 60 min, d'une composition réductrice telle que définie ci-dessus puis, dans une seconde étape, à reformer lesdites liaisons par application d'une composition oxydante ou, éventuellement, en laissant agir l'oxygène de l'air.

De préférence, dans le cas de la permanente, on applique une composition réductrice telle que définie ci-dessus sur des cheveux mouillés préalablement enroulés sur des rouleaux ayant de 4 à 20 mm de diamètre, la composition pouvant, éventuellement, être appliquée au fur et à mesure de l'enroulage des cheveux; on laisse ensuite agir la composition réductrice pendant un temps de 5 à 60 minutes, de préférence de 5 à 30 minutes, puis on rince abondamment ; après quoi on applique, sur les cheveux enroulés, une composition oxydante permettant de reformer les liaisons disulfures de la kératine pendant un temps de pose de 2 à 10 minutes. Après avoir enlevé les rouleaux, on rince abondamment la chevelure.

La pose de la composition réductrice peut se faire sous chaleur.

La composition d'oxydation ou oxydante est du type couramment utilisé et contient, par exemple, comme agent oxydant de l'eau oxygénée, un bromate alcalin, un persel, un polythionate ou un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 10. Cette oxydation peut être effectuée immédiatement ou être différée.

La déformation des cheveux selon l'invention peut également consister en un procédé de défrisage ou de décrêpage des cheveux, dans lequel on applique sur les cheveux une composition réductrice selon l'invention, puis l'on soumet les cheveux à une déformation mécanique permettant de les fixer dans leur nouvelle forme, par une opération de lissage des cheveux avec un peigne à larges dents, avec le dos d'un peigne ou à la main. Après un temps de pose de 5 à 60 minutes, en particulier, de 5 à 30 minutes, on procède alors à un nouveau lissage, puis on rince soigneusement et on applique une composition oxydante ou fixatrice telle que définie ci-dessus, qu'on laisse agir pendant environ 2 à 10 minutes, puis on rince abondamment les cheveux.

Dans chacun des procédés décrits ci-dessus, on peut intercaler une étape avec utilisation d'un fer chauffant entre 60 et 220°C, et de préférence entre 120 et 200°C.

Dans les compositions de permanente selon l'invention, l'agent réducteur de formule générale (I) est notamment présent à une concentration comprise entre 0,05 et 30 % et, de préférence, entre 0,1 et 20 % en poids par rapport au poids total de la composition réductrice.

Le pH est obtenu à l'aide d'un agent alcalin tel que, par exemple, l'ammoniaque, la monoéthanolamine, la diéthanolamine, la triéthanolamine, la propanediamine-1,3, un carbonate ou bicarbonate alcalin ou d'ammonium, un carbonate organique tel que le carbonate de guanidine, un hydroxyde alcalin ou à l'aide d'un agent acidifiant tel que par exemple l'acide chlorhydrique, l'acide acétique, l'acide lactique, l'acide oxalique ou l'acide borique.

La composition réductrice peut également contenir, en association, un autre agent réducteur connu, tel que par exemple l'acide thioglycolique, le monothioglycolate de glycérol ou de glycol, la cystéamine et ses dérivés acylés en C1-C4 tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide bêta-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terreux, les N-(mercaptoalkyl)o-hydroxyalkylamides décrits dans la demande de brevet EP 354 835 et les N-mono- ou N,N-dialkylmercapto 4-butyramides décrits dans la demande de brevet EP 368763, les aminomercaptoalkylamides décrits dans la demande de brevet EP 403 267 et les alkylaminomercaptoalkylamides décrits dans la demande de brevet EP 432 000.

Selon un mode de réalisation préféré, la composition réductrice contient également un agent tensioactif de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, et de préférence, comprise entre 0,5 et 10 % en poids par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les brevets français n° 2.598.613 et n° 2.470.596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2.535.730, les polyorganosiloxanes à groupements aminoalkyles modifiés par des groupements alcoxycarbonyalkyles tels que ceux décrits dans le brevet US n° 4.749.732, des polyorganosiloxanes tels que le copolymère polydiméthylsiloxane- polyoxyalkyle du type Diméthicone Copolyol, un polydiméthylsiloxane à groupements terminaux stéaroxy (stéaroxydiméthicone), un copolymère polydiméthylsiloxane- dialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

La composition réductrice peut également contenir d'autres ingrédients traitants tels que des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n°83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO2/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C3-C6 tels que par exemple le propanediol-1,2 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

La composition réductrice se présente essentiellement sous forme aqueuse, notamment sous la forme d'une lotion épaissie ou non, d'une crème ou d'un gel.

La composition réductrice peut être également du type exothermique, c'est-à-dire provoquant un certain échauffement lors de l'application sur les cheveux, ce qui apporte un agrément à la personne qui subit le premier temps de la permanente ou du défrisage.

La composition réductrice selon l'invention peut également contenir un solvant tel que par exemple de l'éthanol, du propanol ou de l'isopropanol ou encore du glycérol, à une concentration maximale de 20 % par rapport au poids total de la composition.

Le véhicule des compositions est, de préférence, de l'eau ou une solution hydroalcoolique d'un alcool inférieur, tel que l'éthanol, l'isopropanol ou le butanol.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables ou d'alcools gras.

On peut également utiliser des liquides ou des gels contenant des agents épaississants tels que des polymères ou des copolymères carboxyvinyliques qui "collent" les cheveux et les maintiennent dans la position lisse pendant le temps de pose.

L'invention concerne enfin des compositions cosmétiques contenant au moins un composé de formule I tel que défini ci-dessus et au moins un composé, choisi parmi les agents tensioactifs et les polymères non ionique, anionique, cationique, amphotère ou zwitterionique. Toutes ces compositions peuvent en outre contenir les additifs mentionnés précédemment.

L'invention concerne également un kit, notamment, pour la déformation permanente des cheveux comprenant, dans un premier compartiment, en tant que composition réductrice, une composition conforme à l'invention comprenant un composé de formule (I) tel que défini ci-dessus et, éventuellement, au moins un composé, choisi parmi les agents tensioactifs et les polymères non ionique, anionique, cationique, amphotère ou zwitterionique, et dans un second compartiment, une composition oxydante.

L'invention pourra être mieux comprise à l'aide de l'exemple non limitatif qui suit et qui constitue un mode de réalisation préférentiel des compositions selon l'invention.

### Exemples

On réalise des compositions réductrices contenant un agent réducteur de formule (1) à (7) définie dans la description. :

Les composés (1) à (7) ont été caractérisés par RMN du proton, spectrométrie de Masse et dosage de la fonction thiol.

### Synthèse du composé (1)

On additionne sous atmosphère d'argon la solution d'éthylènediamine dans le dichlorométhane (10⁻² mole dans 15 ml) à la solution de N-acétylthiolactone de l'homocystéine dans le dichlorométhane (2 10⁻² mole dans 35 ml) sous agitation magnétique et à température ambiante en 10 minutes. Au bout de deux heures le milieu réactionnel se prend en masse, on porte alors deux heures au reflux du solvant, on refroidit et on filtre le précipité. On lave avec du dichlorométhane, on essore le précipité le plus possible et on le sèche à l'évaporateur rotatif on obtient 3.5 grammes de poudre blanche dépourvue d'odeur.

Masse théorique 3.79; rendement 93 %

Les 6 autres exemples ont été synthétisés selon le même mode opératoire.

| Composé numéro | Amine | N-acétylthiolactone de l'homocystéine | Rendement % |
|---|---|---|---|
| (2) | 1-4 diaminobutane 3. 10⁻² mole | 6.10⁻¹ mole | 91,3 |
| (3) | 1-6 diaminohexane 3.10⁻² mole | 6. 10⁻² mole | 92,5 |
| (4) | Diéthylènetriamine 0.18 mole | 0.36 mole | 99 |
| (5) | Trans-1,2 diaminocyclohexane 0.1 mole | 0.2 mole | 74 |
| (6) | 1-3 diaminopropanol 5.10⁻² mole | 0.1 mole | 93.5 |

| Composé numéro | Amine | Butyrothiolactone | Rendement % |
|---|---|---|---|
| (7) | Ethylènediamine 5.10⁻² mole | 0.1 mole | 81 |

## Revendications

1. Utilisation, pour réduire les liaisons disulfure de la kératine, dans une composition réductrice de déformation de la fibre kératinique, notamment à un pH compris entre 6 et 8, de dithiols de formule I, 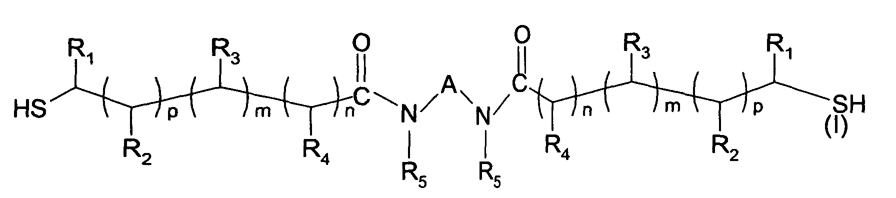 Avec
• n,m, p = 0 ou 1
• R₁, R₂, R₃, R₄ = H, COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
• A = alkylène en C-1 à C-18, saturé ou non, linéaire ou non, pouvant être interrompu par un ou plusieurs cycles aromatiques ou non aromatiques (5, 6 ou 7 chaînons) ou par un ou plusieurs hétéro-atomes ou fonctions tels 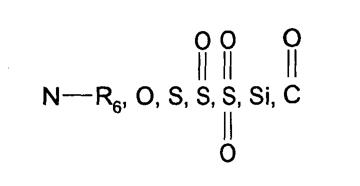 A peut également être représenté par un cycle aromatique ou non aromatique à 5, 6 ou 7 chaînons.
A peut être substitué par un plusieurs groupements COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
• R₅, R₆ = H, alkyl (C-1 à C-4), acyl (C-1 à C-4), alkyl (C-1 à C-4) oxycarbonyl, alkyl (C-1 à C-4) amino-carbonyl, halogéno.

2. Utilisation selon la revendication 1, **caractérisée par le fait que** le composé (I) répond à l'une des formules ci-après:

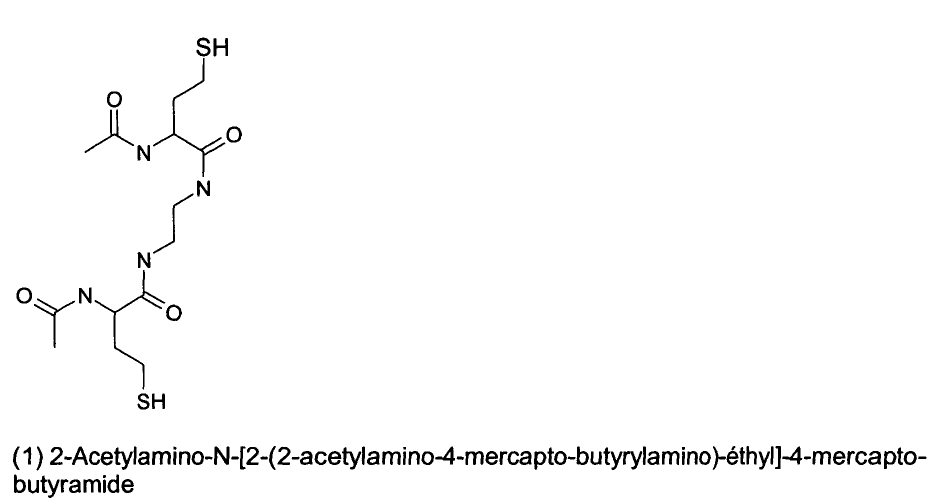

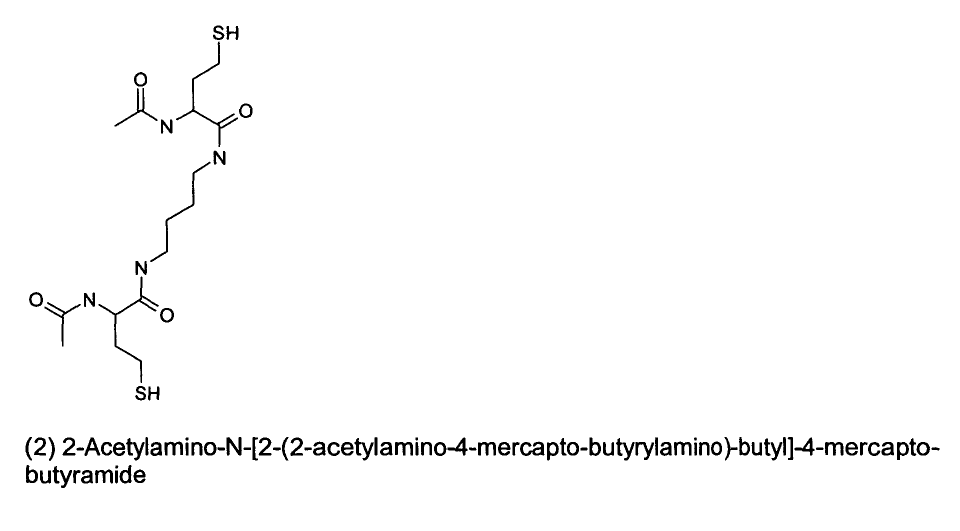

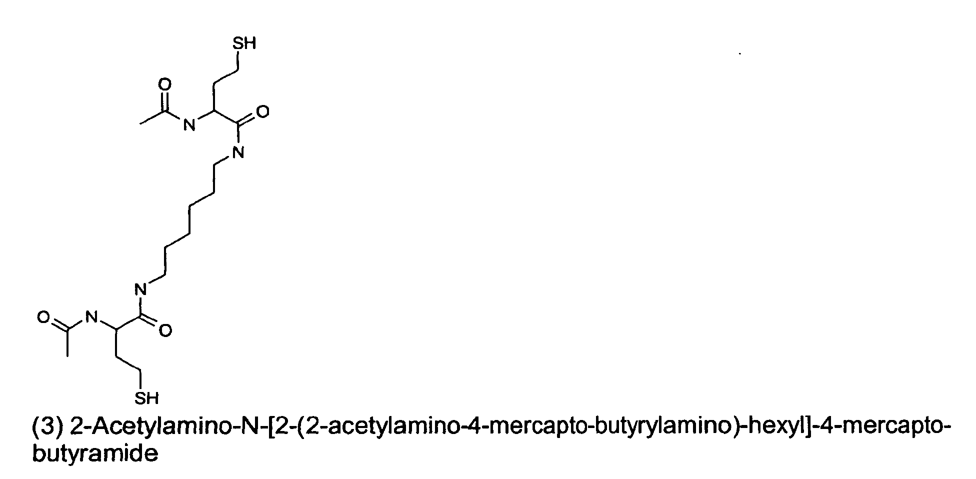

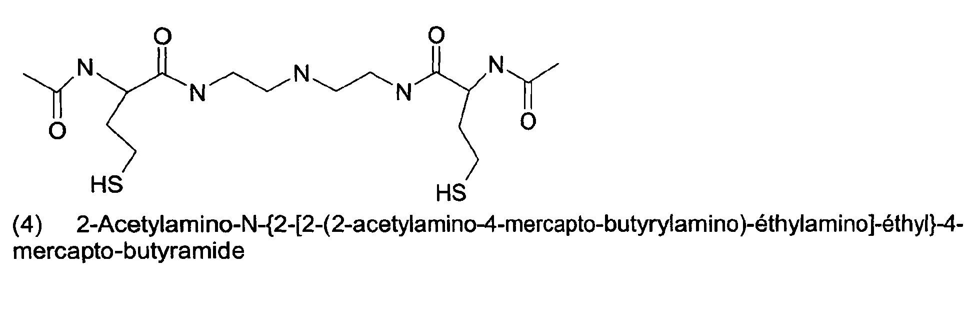

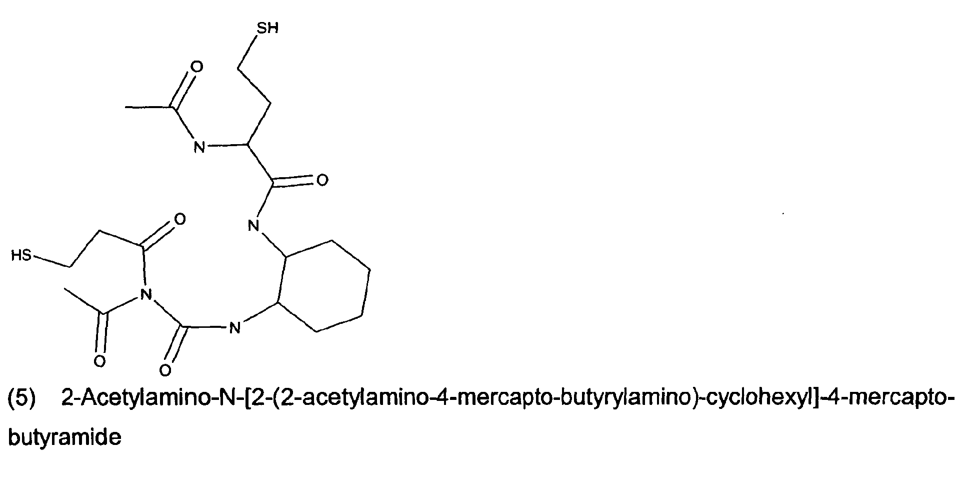

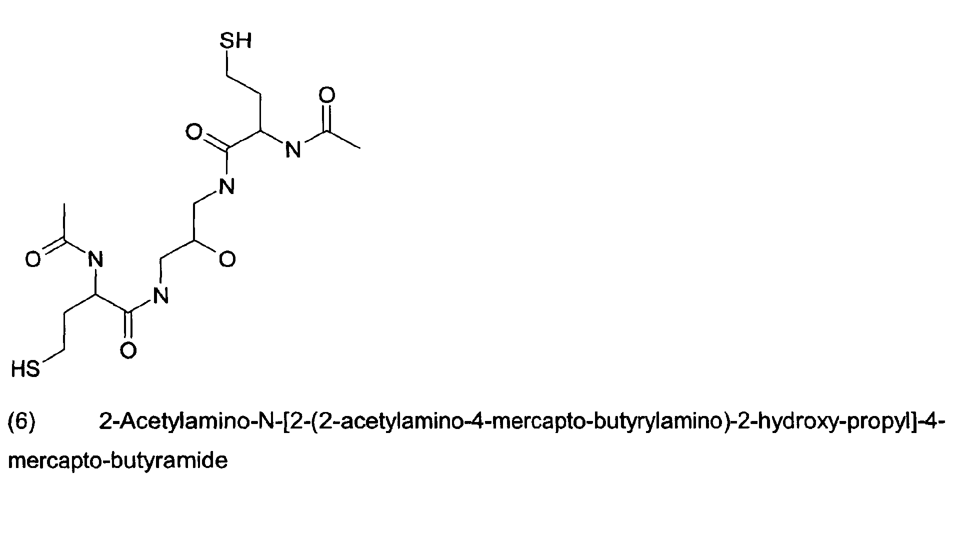

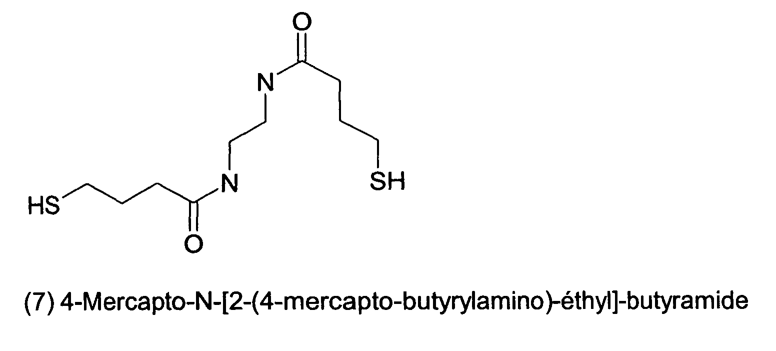

3. Utilisation selon l'une quelconque des revendications précédentes, **caractérisée par le fait que** le pH est compris entre 6,5 et 7,5.

4. Utilisation selon l'une quelconque des revendications précédentes, **caractérisée par le fait que** la déformation est une permanente.

5. Utilisation selon l'une quelconque des revendications précédentes, **caractérisée par le fait que** la déformation est un défrisage.

6. Composition cosmétique comprenant, dans un milieu cosmétiquement acceptable, au moins un dithiol de formule (I) 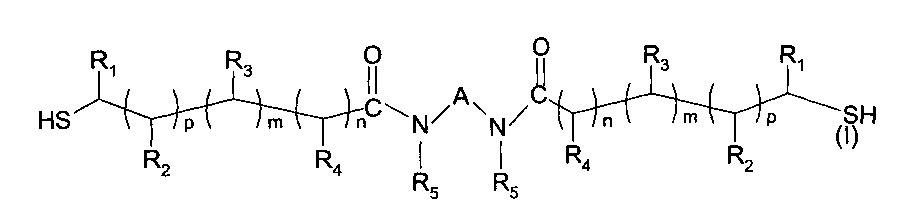 Avec
• n,m,p=0 ou1
• R₁, R₂, R₃, R₄ = H, COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
• A = alkylène en C-1 à C-18, saturé ou non, linéaire ou non, pouvant être interrompu par un ou plusieurs cycles aromatiques ou non aromatiques (5, 6 ou 7 chaînons) ou par un ou plusieurs hétéro-atomes ou fonctions tels 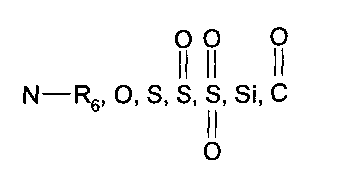 A peut également être représenté par un cycle aromatique ou non aromatique à 5, 6 ou 7 chaînons.
A peut être substitué par un plusieurs groupements COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
• R₅, R₆ = H, alkyl (C-1 à C-4), acyl (C-1 à C-4), alkyl (C-1 à C-4) oxycarbonyl, alkyl (C-1 à C-4) amino-carbonyl, halogéno.

7. Composition cosmétique selon la revendication 6, **caractérisée par le fait que** le composé (I) répond à l'une des formules ci-après:

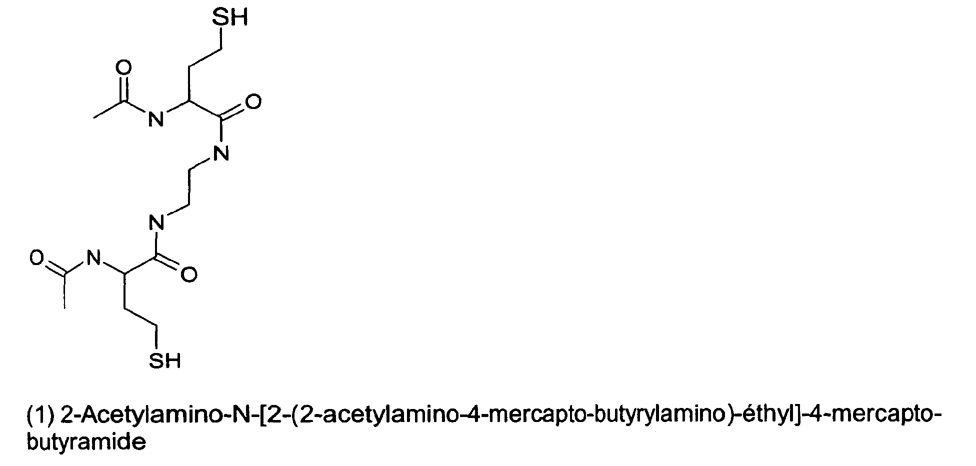

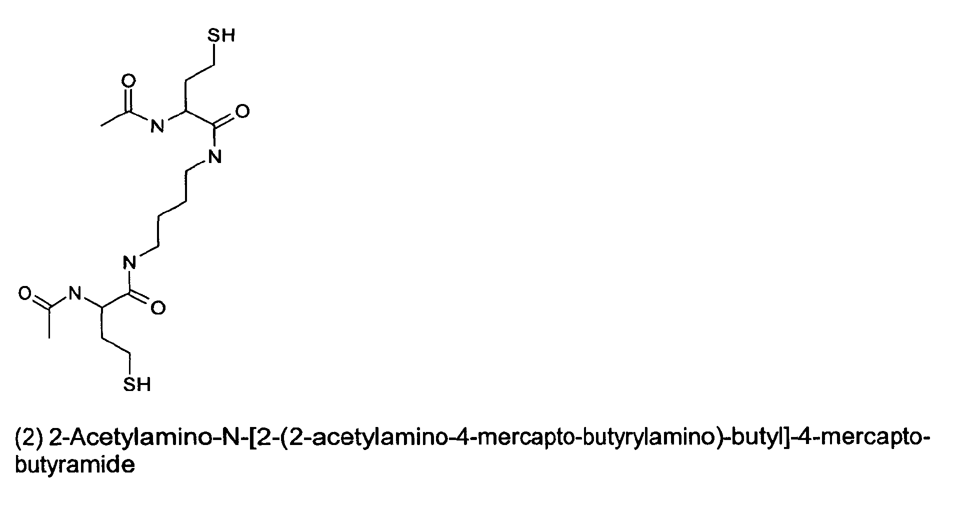

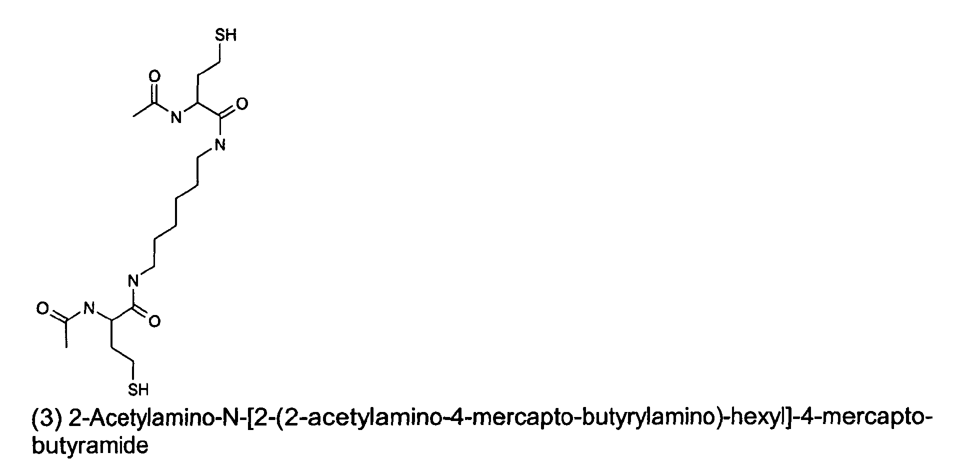

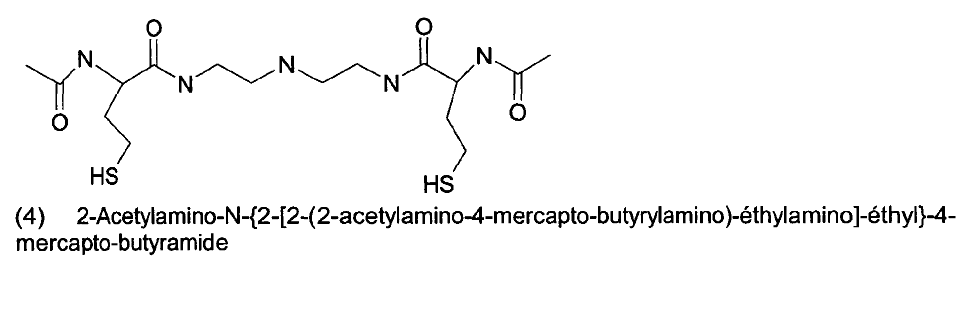

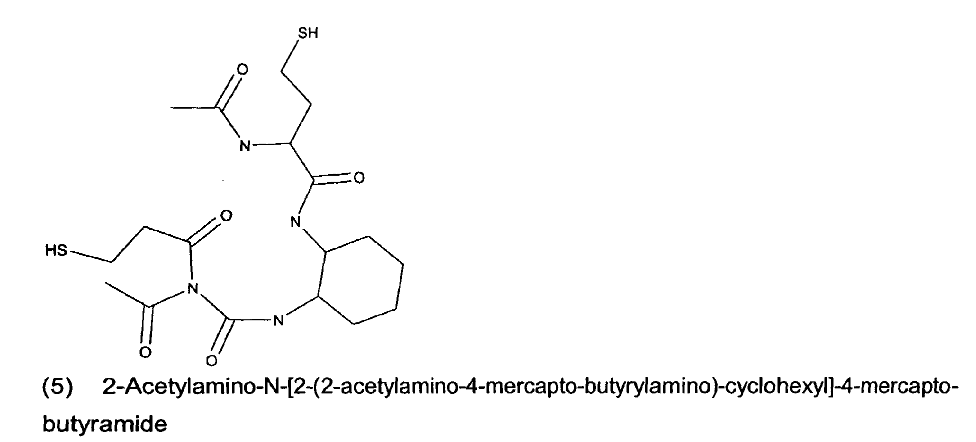

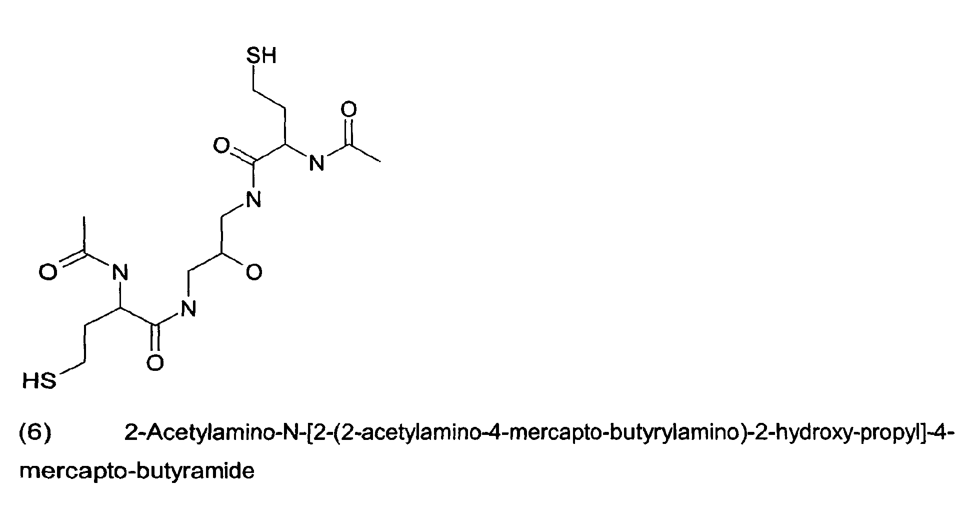

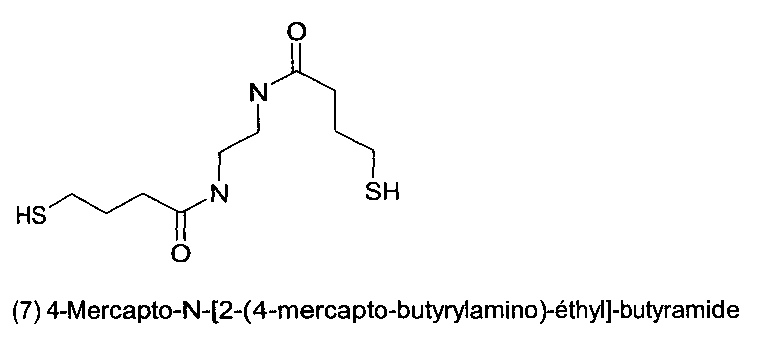

8. Composition selon l'une quelconque des revendications précédentes 6 ou 7, **caractérisée par le fait que** l'agent réducteur de formule générale (I) est présent à une concentration comprise entre 0,05 et 30 % et, de préférence, entre 0,1 et 20 % en poids par rapport au poids total de la composition réductrice.

9. Composition selon l'une quelconque des revendications précédentes 6 à 8, **caractérisée par le fait qu'**elle contient, en outre, un additif choisi parmi un autre agent réducteur connu, un agent tensioactif de type non-ionique, anionique, cationique ou amphotère, un agent traitant de nature cationique, anionique, non-ionique ou amphotère, des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires, des parfums et des conservateurs.

10. Composition cosmétique selon l'une quelconque des revendications 6 à 9, contenant au moins un composé de formule I et au moins un composé, choisi parmi les agents tensioactifs et les polymères non ionique, anionique, cationique, amphotère ou zwitterionique.

11. Procédé de déformation permanente des cheveux, **caractérisé par le fait qu'**il comprend l'application d'une composition réductrice comprenant, en tant qu'agent réducteur, un composé de formule (1).
